(19) Europäisches Patentamt
European Patent Office
Office européen des brevets

(11) Publication number: **0 112 172 B1**

(12) **EUROPEAN PATENT SPECIFICATION**

(45) Date of publication of patent specification: **05.08.87**

(51) Int. Cl.$^4$: **C 07 C 68/00**, C 07 C 69/96

(21) Application number: **83307633.4**

(22) Date of filing: **15.12.83**

(54) **Process for the production of dihydrocarbyl carbonates, their use as fuels additives.**

(30) Priority: **15.12.82 GB 8235694**
**09.09.83 GB 8324216**

(43) Date of publication of application:
**27.06.84 Bulletin 84/26**

(45) Publication of the grant of the patent:
**05.08.87 Bulletin 87/32**

(84) Designated Contracting States:
**BE DE FR GB IT NL SE**

(56) References cited:
**EP-A-0 038 984**

(73) Proprietor: **The British Petroleum Company p.l.c.**
**Britannic House Moor Lane**
**London EC2Y 9BU (GB)**

(72) Inventor: **Morris, George Ernest**
**The British Petroleum Co. p.l.c. Chertsey Road**
**Sunbury-on-Thames Middlesex, TW16 7LN (GB)**

(74) Representative: **Fawcett, Richard Fennelly et al**
**BP INTERNATIONAL LIMITED Patents Division**
**Chertsey Road**
**Sunbury-on-Thames Middlesex, TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 112 172 B1

**Description**

The present invention relates to a process for the production of dihydrocarbyl carbonates, their use as additives in internal combustion engine fuels and fuel compositions containing them.

Dihydrocarbyl carbonates have a number of actual and potential uses, for example dialkyl carbonates as solvents and diphenyl carbonate as an intermediate in the production of aromatic polycarbonates (high performance thermoplastic resins), monoisocyanates (pharmaceuticals and pesticides intermediates), diisocyanates (monomers for polyurethane elastomers) and carbamates (pesticides). Their use as additives to liquid hydrocarbon fuel mixtures has also been proposed.

Conventionally, they are produced by reacting phosgene with alcohols or phenols. Alkyl carbonates can also be produced by reacting saturated aliphatic alcohols with carbon monoxide in the presence as catalyst of palladium or platinum salts (USP 3114762) or an organic copper complex (USP 3846468).

Even more recently the production of dimethyl carbonate by the copper catalysed oxidative carbonylation of methanol by oxygen has been proposed. Furthermore, carbonic diesters are produced as by-products in the production of dialkyl oxalates by reaction of an aliphatic alcohol with carbon monoxide and oxygen under pressure in the presence of a catalyst composed of a mixture of a salt of a platinum group metal and a salt of copper or iron. Of necessity water is formed when an alcohol is reacted with carbon monoxide and molecular oxygen, whether the product be a dialkyl oxalate or a dihydrocarbyl carbonate, or a mixture thereof. It is recognised that the formation of water is undesirable, because it can deactivate the catalyst (see for example USP 4360477), it can complicate product separation and it can depress the yield. For this reason steps have been taken either to remove the water as it is formed in the production of dialkyl oxalates by the addition of a dehydrating agent, for example alkyl orthoformic esters, or to use catalysts which are purportedly not moisture sensitive, for example cupric halides. Moreover, the use of molecular oxygen can raise the problem of explosive mixture formation.

Canadian Patent No. 743559 describes a method of producing a tertiary ester of a carboxylic acid comprising contacting with agitation carbon monoxide and di-t-hydrocarbyl peroxide of the formula $(RR^1R^{11})$ C—O—O—C$(RR^1R^{11})$ wherein R, $R^1$ and $R^{11}$ are hydrocarbyl radicals selected from the group consisting of alkyl, aryl, alkaryl and aralkyl having up to nine carbon atoms, said contacting being conducted at an elevated temperature and carbon monoxide pressure and in the presence of inert organic liquid diluent. The use of transition metal salts as catalysts is said to be desirable for optimum yields of ester. Specific examples of transition metal salts contemplated are said to be halides, carboxylates and nitrates of copper, cobalt, manganese, iron and vanadium.

We have now found that the aforesaid problems associated with the production of dihydrocarbyl carbonates can be substantially overcome by using as the oxidant a dihydrocarbyl peroxide in place of molecular oxygen because the formation of water is thereby avoided and the risk of forming explosive mixtures is diminished. From an economic standpoint the use of a dihydrocarbyl peroxide can be more attractive because desirable alcohols can be formed from the peroxide as the principal by-product of the reaction. Thus, using di-tertiary-butyl peroxide the principal co-product is t-butanol, which is assuming significant importance as a fuels additive.

Accordingly, the present invention provides a process for the production of a dihydrocarbyl carbonate which comprises reacting in the liquid phase under substantially anhydrous conditions a dihydrocarbyl peroxide, a primary or secondary alcohol and carbon monoxide in the presence as catalyst of copper in elemental or compound form.

The term 'hydrocarbyl' is used in the commonly accepted sense as denoting the moiety formed by the removal of a hydrogen atom from a hydrocarbon.

Carbon monoxide is available commercially on a large scale. It may be substantially pure or may contain for example such impurities as hydrogen and/or nitrogen. Suitably a substantial excess of carbon monoxide may be employed. Maintenance of an excess may suitably be accomplished by keeping the reaction system under a carbon monoxide pressure, suitably in the range from 15 to 150 bars, though lower and higher pressures may be employed if desired.

With regard to the dihydrocarbyl peroxide, the dihydrocarbyl radical may suitably be an alkyl, aryl, alkaryl or aralkyl group having up to 9 carbon atoms, and may be the same or different. As the dihydrocarbyl peroxide, there may be used any suitable peroxide, such as for example di-cumyl peroxide, and di-tertiary-butyl peroxide (DTBP), of which DTBP is preferred. As hereinbefore mentioned, by using a peroxide in place of oxygen, the risk of forming explosive mixtures is substantially reduced and the use of dehydrating agents can be avoided because no water is formed in the reaction. DTBP may readily be obtained, for example, by reacting tertiary-butyl alcohol with tertiary-butyl hydroperoxide, which in turn may readily be obtained by oxidation of isobutane. A suitable process for producing DTBP is described in US Patent No 2862973.

As the catalyst there is used copper in elemental or compound form. Although the copper may be added as the elemental metal, suitably in finely divided form, it is preferred to add it in the form of a compound of the metal, suitably as a salt, such as a halide or alkoxide, or a complex. It is preferred to add the copper in compound form and even more preferably as a copper (I) compound. Of the copper (I) compounds the halides, for example copper (I) chloride or copper (I) bromide, are preferred.

The primary or secondary alcohol reactant may be a simple alkanol, eg methanol, ethanol and

propanol; a glycol, eg ethylene glycol; a polyalkylene glycol, eg polyethylene glycol or an aryl alcohol, eg benzyl alcohol. Furthermore, mixtures may be employed if so desired. Preferably an alkanol is employed and most preferably the alkanol is methanol, ethanol or isopropanol, which are commercially available on a large scale.

Although trace amounts of impurities, for example at the levels obtaining in commercially available materials, may be tolerated, substantial amounts of water should be avoided.

It is preferred to employ a catalyst promoter. Suitable promoters include heterocyclic aromatic nitrogen compounds, such as pyridine or a derivative thereof. Examples of suitable promoters which may be used in the process of the invention include pyridine, 2,6-dimethyl pyridine and 2-methyl-5-ethyl pyridine. The heterocyclic aromatic nitrogen compound may be added independently or combined with the catalyst in the form of a complex therewith. Thus pyridine may suitably be added in the form of pyridine copper methoxy chloride.

A diluent may also be employed if so desired. Suitably the diluent may be an aromatic hydrocarbon solvent, such as toluene. Alternatively, the heterocyclic aromatic nitrogen compound, when used as a promoter, may also serve as a diluent.

As regards the reaction conditions, the carbon monoxide partial pressure has been mentioned hereinbefore. The temperature may suitably be above ambient temperature. The actual temperature employed will depend, amongst other factors, upon the amount of catalyst and promoter, if any, used. Generally, the rate of the reaction will increase with increasing catalyst and promoter concentration. Within any constraint imposed by controlling the reaction temperature, for the reaction is highly exothermic, the copper catalyst may be employed in an amount up to and beyond stoichiometric and there is no restriction on the amount of promoter which may be used. However, it will usually be found convenient to add copper in an amount in the range from 1 to 10 mole % (calculated as copper (I) chloride) based on the number of moles of peroxide present, at which concentration the reaction temperature may suitably be greater than 50°C, for example in the range from 80 to 150°C.

The reaction in its simplest form is thought to be represented by the following equation:—

$$R^1{-}O{-}O{-}R^{11}+2R^{111}OH+CO\rightarrow R^{111}{-}O{-}\overset{\displaystyle O}{\overset{\|}{C}}{-}O{-}R^{111}+R^1OH+R^{11}OH \qquad (A)$$

wherein $R^1$ and $R^{11}$ are independently alkyl, aryl, alkaryl or aralkyl groups having up to 9 carbon atoms and $R^{111}$ is the hydrocarbyl group of a primary or secondary alcohol as hereinbefore described. It will be appreciated by those skilled in the art that the dihydrocarbyl peroxide may react directly with carbon monoxide according to the equation:—

$$R^1{-}O{-}O{-}R^{11}+CO\rightarrow R^1{-}O{-}\overset{\displaystyle O}{\overset{\|}{C}}{-}O{-}R^{11} \qquad (B)$$

The extent to which this reaction also occurs will depend to some extent on the type of reactant and the reaction conditions employed.

The process may be operated batchwise or continuously, preferably continuously. The products may be separated in conventional manner. In this respect the absence of water can considerably simplify the separation.

In a particular embodiment the present invention provides a process for the production of a mixture comprising t-butanol and a dialkyl carbonate wherein the alkyl groups are independently either methyl, ethyl or isopropyl which process comprises reacting in the liquid phase under substantially anhydrous conditions at elevated temperature di-tertiary-butyl peroxide, one or more of methanol, ethanol or isopropanol and carbon monoxide in the presence as catalyst of a copper compound and a promoter which is pyridine or a derivative thereof.

Preferably methanol, carbon monoxide and di-tertiary-butyl peroxide are reacted to form a mixture comprising t-butanol and dimethyl carbonate.

The mixture comprising t-butanol and dialkyl carbonate may be recovered and separated into individual components by conventional methods, for example by distillation. The t-butanol may thereafter be dehydrated in known manner to form isobutene and the isobutene hydrogenated in known manner to form isobutane, which may then be recycled as feed to the reaction producing DTBP. Alternatively, the t-butanol may be used as a fuels supplement, as may also the dialkyl carbonate.

The invention is now illustrated by the following Examples.

Example 1

An autoclave was charged with methanol (8.9 gm), di-tertiary-butyl peroxide (20.4 gm), cuprous chloride (0.28 gm) and pyridine (2.2 gm). Carbon monoxide gas was introduced to a pressure of 46 bar at ambient temperature. The mixture was heated, with stirring to 92°C and maintained at this temperature for 18 h. During this time further carbon monoxide gas was introduced as necessary to maintain a pressure

between 47 and 51 bar. After cooling to ambient temperature the mixture was analysed by gas chromatography and $^1H$ n.m.r. The major component was tertiary butanol and no residual di-tertiary butyl peroxide was detected. The mixture also contained dimethyl carbonate (11.1 gm), methanol and pyridine as well as small amounts of other compounds among which were identified acetone and methyl acetate.

Example 2

An autoclave was charged with methanol (32 ml), di-tertiary-butyl peroxide (14.5 ml) and pyridine copper methoxy chloride (0.85 gm). Carbon monoxide gas was introduced to a pressure of 75 bar at 22°C. The mixture was heated to 120°C with stirring, over a period of 9 minutes. An exothermic reaction took place, the temperature of the mixture reaching a maximum value of 188°C after a further 90 seconds while the recorded pressure decreased. The reaction mixture returned to 120°C during 20 minutes and no further gas uptake occurred. After cooling to room temperature the $^1H$ n.m.r. of the product mixture showed a molar ratio of methanol:tertiary butanol:dimethyl carbonate of 4.4:1:0.41, as well as traces of acetone and methyl acetate.

Example 3

An autoclave was charged with methanol (32 ml), di-tertiary butyl peroxide (14.5 ml), pyridine (1 ml) and cuprous chloride (0.4 gm). Carbon monoxide gas was introduced to a pressure of 37.5 bar, then hydrogen gas to give a total pressure of 67 bar at 24°C. The mixture was heated to 120°C, with stirring, over a period of 8 minutes. An exothermic reaction took place, the temperature of the mixture reaching a maximum value of 160°C after a further 90 seconds. The reaction mixture returned to 120°C during 25 minutes and no further uptake of gas occurred. After cooling to room temperature the $^1H$ n.m.r. of the product mixture showed a molar ratio of methanol:tertiary butanol:dimethyl carbonate of 3.9:1:0.37 as well as traces of acetone and methyl acetate.

Example 4

An autoclave was charged with methanol (9.0 gm), di-tertiary butyl peroxide (20.4 gm), cuprous chloride (0.28 gm) and 2,6-dimethyl pyridine (3.1 gm). Carbon monoxide gas was introduced to a pressure of 46 bar at ambient temperature. The mixture was heated, with stirring to 97°C and maintained at this temperature for 19 h. During this time further carbon monoxide gas was introduced as necessary to maintain the pressure between 46 and 52 bar.

After cooling to ambient temperature the mixture was analysed by gas chromatography and $^1H$ n.m.r. The major component was tertiary butanol and no residual di-tertiary-butyl peroxide was detected. The mixture also contained dimethyl carbonate (11.2 gm), methanol and 2,6-dimethyl pyridine as well as small amounts of other compounds among which were identified acetone and methyl acetate.

Example 5

An autoclave was charged with methanol (9.0 gm), di-tertiary butyl peroxide (20.5 gm) cuprous chloride (0.29 gm) and pyridine (2.2 gm). Hydrogen gas was introduced to a pressure of 22 bar at ambient temperature. Carbon monoxide was then introduced to a total pressure of 46 bar at ambient temperature. The mixture was heated with stirring to 92°C and maintained at this temperature for 15 h. Further carbon monoxide gas was introduced as necessary to maintain a pressure between 41 and 50 bar.

After cooling to ambient temperature the mixture was analysed by gas chromatography and $^1H$ n.m.r. The major component was tertiary butanol and no residual di-tertiary-butyl peroxide was detected. The mixture also contained dimethyl carbonate (10.7 gm), methanol and pyridine as well as small amounts of other compounds among which were identified acetone and methyl acetate.

Example 6

An autoclave was charged with methanol (9.0 gm), di-tertiary-butyl peroxide (20.5 gm) and pyridine copper methoxy chloride (0.59 gm). Carbon monoxide gas was introduced to a pressure of 45 bar at ambient temperature. The mixture was heated, with stirring to 92°C and maintained at this temperature for 12 h. During this time further carbon monoxide gas was introduced as necessary to maintain a pressure between 40 and 51 bar. After cooling to ambient temperature the mixture was analysed by gas chromatography and $^1H$ n.m.r. The major component was tertiary-butanol and no residual di-tertiary-butyl peroxide was detected. The mixture also contained dimethyl carbonate (8.6 gm) and methanol as well as small amounts of other compounds among which were identified acetone and methyl acetate.

Example 7

An autoclave was charged with ethanol (16.6 gm), di-tertiary-butyl peroxide (17.5 gm) cuprous chloride (0.24 gm) and pyridine (0.6 gm). Carbon monoxide gas was introduced to a pressure of 72 bar at ambient temperature. The mixture was heated with stirring to 100°C and maintained at this temperature for 18 h. During this time further carbon monoxide gas was introduced as necessary to maintain a pressure between 88 and 99 bar. After cooling to ambient temperature the mixture was analysed by gas chromatography and $^1H$ n.m.r. The major component was tertiary-butanol. The mixture also contained diethyl carbonate (8.2 gm), ethanol and pyridine as well as small amounts of other unidentified components.

**Claims**

1. A process for the production of a dihydrocarbyl carbonate which comprises reacting in the liquid phase under substantially anhydrous conditions a dihydrocarbyl peroxide, a primary or secondary alcohol and carbon monoxide in the presence as catalyst of copper in elemental or compound form.

2. A process according to claim 1 wherein the hydrocarbyl groups of the dihydrocarbyl peroxide are independently either alkyl, aryl, alkaryl or aralkyl groups having up to nine carbon atoms.

3. A process according to claim 2 wherein the dihydrocarbyl peroxide is di-tertiary-butyl peroxide.

4. A process according to any one of the preceding claims wherein copper is added in the form of a copper (I) compound.

5. A process according to claim 4 wherein the copper (I) compound is a halide.

6. A process according to any one of the preceding claims wherein the alcohol is an alkanol which is either methanol, ethanol or isopropanol.

7. A process according to any one of the preceding claims wherein there is added an heterocyclic aromatic nitrogen compound.

8. A process according to claim 7 wherein the heterocyclic aromatic nitrogen compound is either pyridine or a derivative thereof.

9. A process according to claim 8 wherein di-tertiary-butyl peroxide, carbon monoxide and one or more of methanol, ethanol or isopropanol are reacted in the presence as catalyst of a copper compound to produce a mixture comprising t-butanol and a dialkyl carbonate wherein the alkyl groups are independently either methyl, ethyl or isopropyl.

10. A process according to claim 9 wherein di-tertiary-butyl peroxide, carbon monoxide and methanol are reacted to produce a mixture comprising t-butanol and dimethyl carbonate.

11. A process according to either claim 9 or claim 10 wherein a mixture comprising t-butanol, a dialkyl carbonate wherein the alkyl groups are independently either methyl, ethyl or isopropyl, and all or part of the unreacted methanol, ethanol or isopropanol is recovered.

**Patentansprüche**

1. Verfahren zur Herstellung eines Dihydrocarbylcarbonats durch Umsetzung in flüssiger Phase unter im wesentlichen wasserfreien Bedingungen eines Dihydrocarbylperoxids mit einem primären oder sekundären Alkohol und Kohlenmonoxid in Anwesenheit von Kupfer in elementarer Form oder als Kupferverbindung als Katalysator.

2. Verfahren gemäß Anspruch 1, wobei die Hydrocarbylgruppen des Dihydrocarbylperoxids unabhängig voneinander Alkyl-, Aryl-, Alkaryl- oder Aralkyl-Gruppen mit bis zu neun Kohlenstoffatomen sind.

3. Verfahren gemäß Anspruch 2, wobei das Dihydrocarbylperoxid Di-tert.-Butylperoxid ist.

4. Verfahren gemäß einem der vorgehenden Ansprüche, worin Kupfer zugesetzt wird in Form einer Kupfer(I)-Verbindung.

5. Verfahren gemäß Anspruch 4, worin die Kupfer(I)-Verbindung ein Halogenid ist.

6. Verfahren gemäß einem der vorgehenden Ansprüche, worin der Alkohol ein Alkanol ist und zwar entweder Methanol, Ethanol oder Isopropanol.

7. Verfahren gemäß einem der vorgehenden Ansprüche, worin eine heterozyklische, aromatische Stickstoffverbindung zugesetzt wird.

8. Verfahren gemäß Anspruch 7, worin die heterozyklische, aromatische Stickstoffverbinding entweder Pyridin oder ein Derivat davon ist.

9. Verfahren gemäß Anspruch 8, worin Di-tert.-Butylperoxid, Kohlenmonoxid und eine oder mehrere von Methanol, Ethanol oder Isopropanol in Anwesenheit einer Kupferverbindung als Katalysator umgesetzt werden zur Herstellung einer Mischung enthaltend Tert.-Butanol und ein Dialkylcarbonat, worin die Alkylgruppen unabhängig voneinander entweder Methyl, Ethyl oder Isopropyl sind.

10. Verfahren gemäß Anspruch 9, worin Di-tert.-Butylperoxid, Kohlenmonoxid und Methanol umgesetzt werden zur Herstellung einer Mischung enthaltend Tert.-Butanol und Dimethylcarbonat.

11. Verfahren gemäß Anspruch 9 oder 10, worin eine Mischung enthaltend Tert.-Butanol, ein Dialkylcarbonat, worin die Alkylgruppen unabhängig voneinander Methyl, Ethyl oder Isopropyl sind und das gesamte oder ein Teil des nicht umgesetzten Methanols, Ethanols oder Isopropanols wiedergewonnen werden.

**Revendications**

1. Procédé pour produire un carbonate de dihydrocarbyle, qui comprend la réaction, en phase liquide dans des conditions essentiellement anhydres, d'un peroxyde de dihydrocarbyle, d'un alcool primaire ou secondaire et du monoxyde de carbone, en présence, comme catalyseur, de cuivre sous forme élémentaire ou sous forme d'un composé.

2. Procédé selon la revendication 1, dans lequel les groupes hydrocarbyles du peroxyde de

dihydrocarbyle sont, indépendamment, des groupes alkyles, aryles, alkaryles ou aralkyles ayant jusqu'à 9 atomes de carbone.

3. Procédé selon la revendication 2, dans lequel le peroxyde de dihydrocarbyle est le peroxyde de ditertio-butyle.

4. Procédé selon l'une quelconque des revendications précédentes, dans lequel le cuivre est ajouté sous la forme d'un composé de cuivre-(I).

5. Procédé selon la revendication 4, dans lequel le composé de cuivre-(I) est un halogénure.

6. Procédé selon l'une quelconque des revendications précédentes, dans lequel l'alcool est un alcanol qui est le méthanol, l'éthanol ou l'isopropanol.

7. Procédé selon l'une quelconque des revendications précédentes, dans lequel on ajoute un composé hétérocyclique à azote aromatique.

8. Procédé selon la revendication 7, dans lequel le composé hétérocyclique à azote aromatique est la pyridine ou un de ses dérivés.

9. Procédé selon la revendication 8, dans lequel on fait réagir le peroxyde de di-tertio-butyle, le monoxyde de carbone et un ou plusieurs alcools choisis parmi le méthanol, l'éthanol et l'isopropanol, en opérant en présence, comme catalyseur, d'un composé du cuivre pour produire un mélange comprenant du tertio-butanol et un carbonate de dialkyle, dans lequel les groups alkyles sont, indépendamment, les groupes méthyle, éthyle ou isopropyle.

10. Procédé selon la revendication 9, dans lequel on fait réagir le peroxyde de di-tertio-butyle, le monoxyde de carbone et le méthanol pour produire un mélange comprenant du t-butanol et le carbonate de diméthyle.

11. Procédé selon la revendication 9 ou la revendication 10, dans lequel on récupère un mélange comprenant du tertio-butanol, un carbonate de dialkyle dans lequel les groupes alkyles sont, indépendamment, un groupe méthyle, éthyle ou isopropyle, et l'on récupère la totalité ou une partie du méthanol, de l'éthanol ou de l'isopropanol n'ayant pas réagi.